# EUROPEAN PATENT SPECIFICATION

(11) **EP 3 858 309 B1**
(45) Date of publication and mention of the grant of the patent: **11.12.2024**
(21) Application number: 19866704.0
(22) Date of filing: 16.07.2019
(51) Int. Cl.: A61F 13/15, A61F 13/47, A61F 13/472, A61F 13/551, A61F 13/84

(54) **PACKAGING SHEET AND PACKAGED ABSORBENT ARTICLE**
VERPACKUNGSFOLIE UND VERPACKTER ABSORBIERENDER ARTIKEL
FEUILLE D'EMBALLAGE ET ARTICLE ABSORBANT EMBALLÉ

(30) Priority: 27.09.2018 JP 2018182549
(43) Date of publication of application: 04.08.2021
(73) Proprietor: DAIO PAPER CORPORATION, Shikokuchuo-shi Ehime 799-0492 (JP)
(72) Inventor: TAGOMORI, Junta, Sakura-shi, Tochigi 329-1411 (JP)
(74) Representative: Epping - Hermann - Fischer
(86) International application number: PCT/JP2019/027854
(87) International publication number: WO 2020/066230

(56) References cited:
- EP-A1- 2 623 077
- WO-A1-2013/080757
- JP-A- 2007 521 117
- US-A1- 2006 025 739
- US-A1- 2012 310 201

## Description

### TECHNICAL FIELD

The present invention relates to a wrapping sheet and a wrapped absorbent article.

### BACKGROUND OF THE INVENTION

Generally, absorbent articles such as sanitary napkins, panty liners, and incontinence pads are typically provided in a wrapped form by a wrapping sheet for hygiene and portability purposes. As such wrapped absorbent articles, it is known that the absorbent articles are mounted on an elongated wrapping sheet, the wrapping sheet is wound in a lengthwise direction and triple folded to wrap the absorbent articles, and the edges in widthwise direction are sealed with a heat seal or the like (for example, FIG. 8 of Patent Document 1). In a triple-wound fold, the wrapping sheet is laminated and folded so that the inner surface side of one end area of the sheet contacts the outer surface side of the other end area of the sheet.

### RELATED-ART DOCUMENT

### Patent Document

Patent Document 1: Japanese Patent No. 6169393
Further, documents WO 2013/080757 A1, US 2006/025739 A1, EP 2 623 077 A1 and US 2012/310201 A1 refer to absorbent articles.

### SUMMARY OF THE INVENTION

### Problems to be Solved by the Invention

It is not preferable that absorbent articles are in plain sight from others. Therefore, it is preferable that an absorbent article wrapped by a wrapping sheet does not look obvious as an absorbent article at first glance. Accordingly, to provide a shielding function to a wrapping sheet, a colorant layer is often formed by printing or the like on the surface of the wrapping sheet.

However, when a colorant layer is formed on an outer surface side of a wrapping sheet and the wrapping sheet is tri-folded and sealed, the colorant layer would be placed between the outer surface side of one end region and the inner surface side of the other end region that overlaps the outer surface. As a result, a sealability may be reduced, and a seal portion tends to be opened.

In view of the foregoing, one aspect of the present invention is to provide a wrapping sheet for an absorbent article having a shielding function and constituting a wrapped absorbent article with high sealability.

### Means for Solving the Problem

In one aspect of the present invention, a wrapping sheet for an absorbent article which is configured to be wrapped around the absorbent article such that a first region including one end of the wrapping sheet in a lengthwise direction overlaps a second region including an opposite end of the wrapping sheet in the lengthwise direction, and which is provided with seal portions at edges of the wrapping sheet in a widthwise direction that are openably sealed to enable wrapping of the absorbent article, wherein a colorant layer is formed on an exposed region exposed to an outside when the absorbent article is wrapped, such that the absorbent article is not easily visible from the outside, and wherein a portion of the seal portions between the first region and the second region does not have the colorant layer, or has the colorant layer at an area ratio of 30% or less. The colorant layer is not present in portions near the one end in the lengthwise direction of overlapping seal portions.The colorant layer is formed with the area ratio of greater than 30% and 100% or less in the exposed region.

### Effects of the Invention

According to one aspect of the invention, a wrapping sheet for an absorbent article having a shielding function and constituting a wrapped absorbent article with high sealability can be provided.

### BRIEF DESCRIPTION OF THE DRAWINGS

FIG. 1 illustrates an unfolded state of a wrapping sheet viewed from an outer surface side of the wrapping sheet of the invention;
FIG. 2 illustrates a wrapped absorbent article in which an absorbent article is wrapped in the wrapping sheet of FIG. 1;
FIG. 3 is a cross-sectional view taken along line I-I of FIG. 2;
FIG. 4 is a cross-sectional view taken along line II-II of FIG. 2;
FIG. 5 is a partially enlarged view of FIG. 1;
FIG. 6 illustrates modification example of a wrapped absorbent article;
FIG. 7 illustrates an unfolded state of a wrapping sheet viewed from an outer surface side of the wrapping sheet of another embodiment of the invention;
FIG. 8 illustrates a wrapped absorbent article in which the absorbent article is wrapped in the wrapping sheet of FIG. 7;
FIG. 9 is a cross-sectional view taken along line III-III of FIG. 8; and
FIG. 10 is a cross-sectional view taken along line IV-IV of FIG. 8.

### DETAILED DESCRIPTION OF THE INVENTION

Embodiments of the present invention will be described in detail with reference to the drawings. In each drawing, unless otherwise noted, the description of the same or corresponding structure may be omitted with the same reference numerals. The drawings are schematic to aid in understanding the invention.

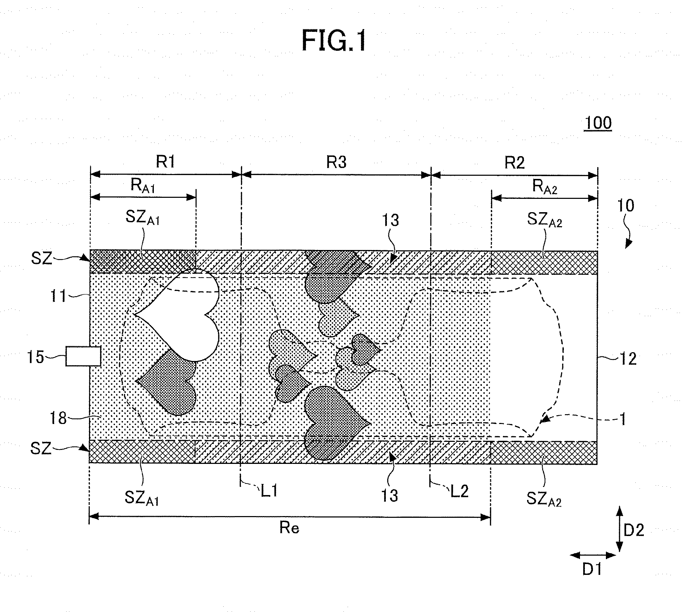

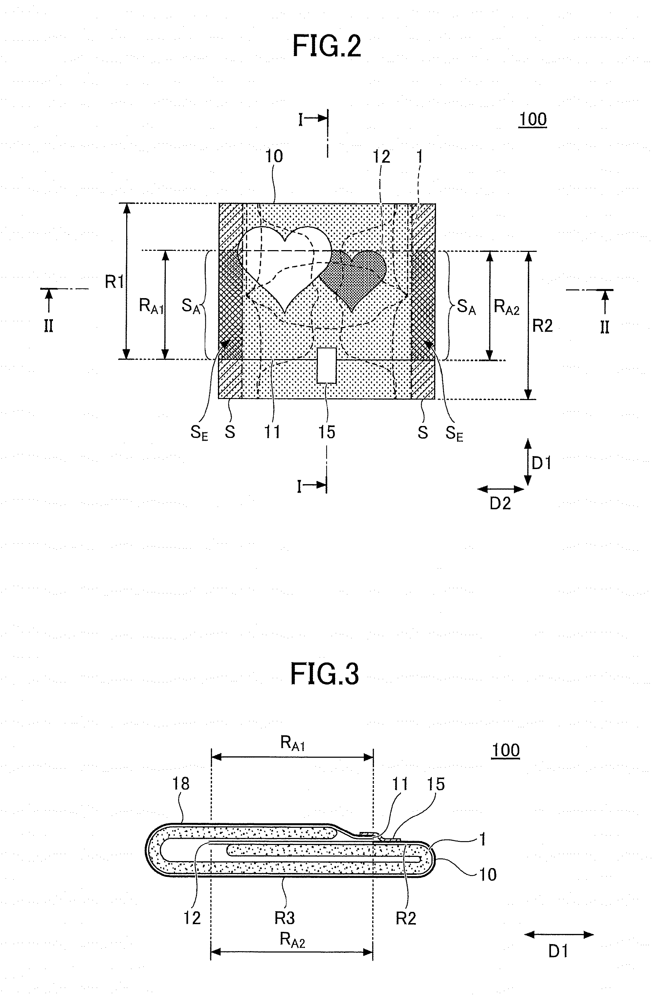

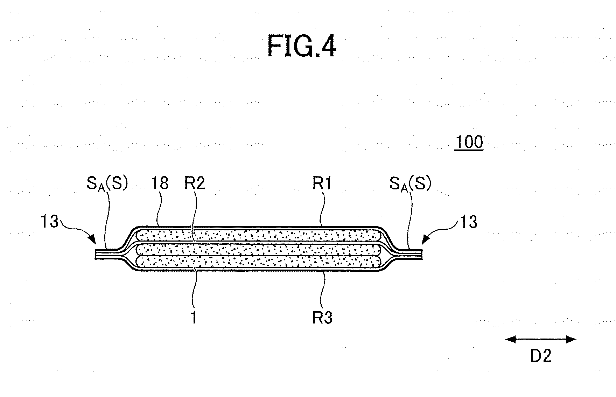

FIG. 1 illustrates one embodiment of a wrapping sheet 10 for an absorbent article. FIG. 1 illustrates a view from the outer surface side of the unfold wrapping sheet (the opposite side of the surface on which the absorbent article 1 is placed) where an absorbent article 1 is placed on the inner surface side of the unfolded wrapping sheet 10. FIG. 1 also illustrates an unfolded state of the wrapped absorbent article 100. Also FIG. 2 illustrates a wrapped absorbent article wrapped with the wrapping sheet 10. That is, FIG. 2 illustrates a plan view of the wrapped absorbent article 100. Further, FIG. 3 illustrates a cross-sectional view of I-I line in FIG. 2, and FIG. 4 illustrates a cross-sectional view of II-II line in FIG. 2.

As illustrated in FIG. 1, the wrapping sheet 10 has an elongated rectangular shape. The first direction D1 in FIG. 1 is a lengthwise direction of the wrapping sheet 10, and the direction perpendicular to the first direction D1 is a widthwise direction D2. The size of the wrapping sheet 10 depends on the size and shape of the absorbent article 1 to be wrapped. For example, the length of the first direction D1 may be in the range of 120 to 500 mm and the length of the second direction D2 may be in the range of 75 to 180 mm when the wrapping sheet 10 is unfolded (FIG. 1). When the wrapping sheet is folded (FIG. 2), the length of the first direction D1 may be about 50 to 160 mm.

The wrapping sheet 10 may be a polyolefin film, such as polypropylene or polyethylene, a resin film, such as polyester or polyvinyl alcohol, or a non-woven fabric or laminated non-woven fabric (including a non-woven polylaminated material in which a polymer layer is laminated on one or both surfaces of the non-woven fabric). Resin films are preferably used if a design by printing is desired, and non-woven fabric is preferably used if a texture and soft feel are desired.

The wrapping sheet 10 can wrap the absorbent article 1 by tri-folding after the absorbent article 1 is placed on the inner surface side of the wrapping sheet. That is, the wrapping sheet 10 can cover the absorbent article and enclose the absorbent article in the sheet. The tri-folding refers to a folding method in which the sheet-like body is divided into three regions in one direction and the regions at both ends of the three regions are folded on the same side so that at least partially the regions at both ends are overlapped. In this embodiment, the first region R1, which includes a one end 11 thereof in the lengthwise direction D1, can be folded so as to overlap the second region R2, which includes an opposite end 12 of the lengthwise direction D2. More specifically, the wrapping sheet 10 can be mountain-folded at the second region R2 with the second folding line L2 and then mountain-folded at the first region R1 with the first folding line L1 as illustrated in FIG. 1.

The wrapping sheet 10 is folded with the absorbent article 1 to provide the wrapped absorbent article 100 as illustrated in FIGS. 2 to 4. A tag tape 15 is provided at the one end 11 thereof in the wrapping sheet 10 so that the first region R1 can be secured to the second region R2 by the tag tape 15 after the wrapping sheet 10 is folded with the absorbent article 1. When opened, the wrapped absorbent article 100 can be unfolded by pulling the tag tape 15.

The central region between the first region R1 and the second region R2 is the third region R3. In the first region R1, the region that is overlapped on the second region R2 is designated as the first overlapping region R_{A1}. In the second region R2, the region that overlaps the firth region R1 is designated as the second overlapping region R_{A2}.

In the state immediately after wrapping the absorbent article 1 by tri-folding with the wrapping sheet 10, the edges 13 and 13 of the widthwise direction D2 remain open. By sealing the edges 13 and 13 in the widthwise direction D2 so as to be openable, the absorbent article 1 can be enclosed in the space formed by the packaging sheet 10. This allows the absorbent article 1 to be hygienically carried as the wrapped absorbent article 100, because the absorbent article 1 is not exposed to dust or dirt.
In the illustrated wrapped absorbent article 100, the sealing portions S and S are formed by sealing the both edges 13 and 13 in the widthwise direction D2 so as to be openable (indicated by diagonal lines in FIGS. 2 and 4).

As illustrated in FIG. 4, the seal portions S and S are portions where a plurality of layers formed by folding the wrapping sheet 10 are bonded to each other. Therefore, when three or more layers of the wrapping sheets 10 are laminated, the contacted two layers are bonded to each other.

Note that a seal to be openable refers to a seal to be formed by bonding the sheet so as not to open in normal handling such as carrying before use, but to be openable by hand when used. In addition, although the strength of the seal to be openable is not particularly limited, it is preferable that the wrapping sheet 10 is not easily broken when a user opens the seal.

The seal types of seal portions S and S are not particularly limited as long as the seal can be opened by hand without using any special tool, and may be formed by heat sealing, ultrasonic sealing, or by using an adhesive.

The seal portions S and S may be formed within a range of up to 25 mm from the end of the widthwise direction of the wrapping sheet 10. In addition, the seal portions S and S may be formed within the above-described range with a width of about 5 to 25 mm. Therefore, the seal portions S and S can be formed, for example, at a distance of 20 mm or less from the edge in the widthwise direction. In the embodiment as illustrated, the seal portions S and S are formed in the same width across the lengthwise direction D1 of the wrapping sheet 10 in the state which wraps the absorbent article 1 over a predetermined position from the end of the widthwise direction of the wrapping sheet 10. The width of the seal portions S and S may vary over the lengthwise direction D1.

In the unfolded wrapping sheet 10, the regions in which the seal portions S and S are formed are defined as the seal zone SZ (FIG. 1). At the edge portions 13 and 13 of the widthwise direction of the wrapping sheet 10, the seal zones SZ and SZ extend from the one end 11 to the opposite end 12 thereof in the lengthwise direction D1, as illustrated by diagonal lines in FIG. 1.

The seal portion S (seal zone SZ) may include the portion where the seal processing was actually performed and the surrounding portion where the properties and conditions changed due to the seal processing. For example, when the sealing method is a heat seal, it includes not only the adhesion portion formed by the heat seal, but also portions where the material has changed or has been deformed, such as by thermal denaturation around the adhesion portion.

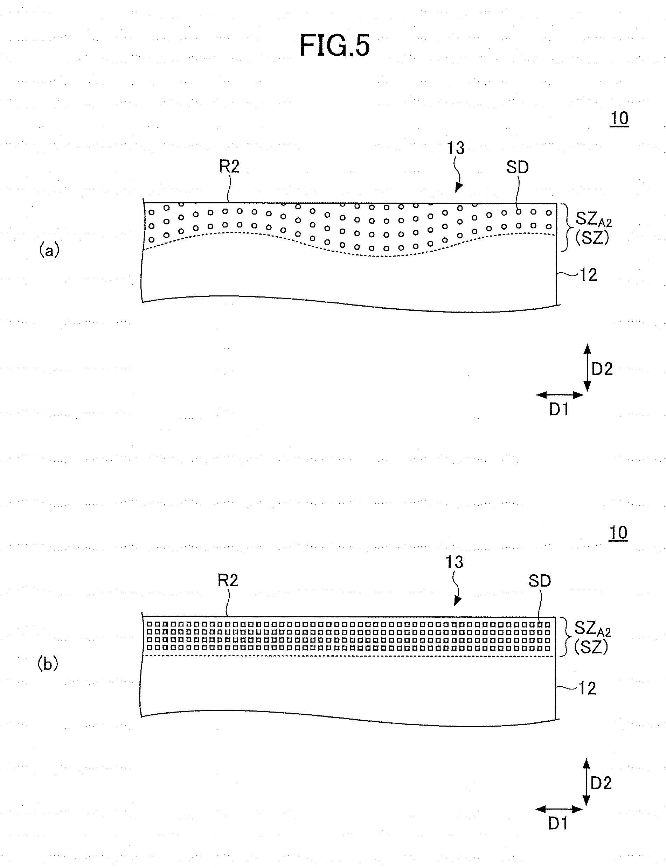

FIG. 5(a) illustrates an enlarged view of the seal zone SZ of the packaging sheet 10 illustrated in FIG. 1. FIG. 5(a) is an enlarged view of the seal zone SZ (seal zone SZ_{A2} etc. will be described in detail later) near the opposite end 12 thereof in the second region R2. The seal zone SZ is a band-like zone having a predetermined width, but from a microscopic viewpoint, as illustrated in FIG. 5(a), a collection of dots-shaped seal processing portions SD is illustrated. The pattern of the seal processing portions SD in this seal zone SZ corresponds to the pattern of the adhesion portion of the seal portions S in the wrapped absorbent article 100.

The pattern of the seal processing portions SD may be discontinuous (FIG. 5(a)) or continuous in the seal zone SZ regardless of the type of seal. For example, the seal processing portions SD may be spread throughout the seal zone SZ. That is, the layers of the wrapping sheets 10 may be bonded throughout the area of the seal portions S. Linear seal portions may also be provided in parallel or meshed.

In the embodiment of Fig. 5(a), the contour of the seal zone SZ (the boundary between the seal zone SZ and the rest of the region) includes a curve that can be perceived as a wavy line during normal use or when viewed from away. By changing the width of the seal zone SZ by using the contour of the seal zone SZ as a wavy line, the design of the seal zone SZ can be improved, and at the same time, it is possible to balance the strength of the seal processing by forming a locally low seal strength portion.

A modification example of the seal zone SZ is illustrated in FIG. 5(b). The seal zone SZ illustrated in FIG. 5(a) is also formed by a collection of dot-shaped seal processing portions SD. However, in the modification example, the shape of the seal processing portions SD in the plan view is square, and the contour of the seal zone SZ is a straight line.

In addition to circular and square shapes, the shape of the seal processing portions SD in the plan view may be elliptical, polygonal (including triangular, quadrilateral other than square, or the like), heart-shaped, drop-shaped, star-shaped, or a part of these shapes.

The entire first region R1 and third region R3 and a portion of the second region R2 of the wrapping sheet 10 form an exposed region Re (FIG. 1). The exposed region Re is a region externally exposed in the state in which the absorbent article 1 is wrapped with the wrapping sheet (e.g., FIGS. 2 and 3). In the exposed region Re, a colorant layer 18 is formed so that the absorbent article 1 is not easily visible from the outside in the state in which the absorbent article 1 is wrapped.

In the present specification, "not easily visible" refers that when a wrapped absorbent article 100 is within one's eye sight, the person does not recognize that the content is the absorbent article 1. Whether the content may or may not be recognized as the absorbent article 1 is not a matter in the specification when the wrapped absorbent article 100 is carefully observed, for example, by holding the wrapped absorbent article 100 against the light.

In general, it is desirable to keep absorbent articles from being visible to others when absorbent articles are carried. Therefore, when carrying absorbent articles when going out, some users try to further wrap wrapped absorbent articles with a cloth or place wrapped absorbent articles in a pouch so that the contents are not known to be absorbent articles. In this embodiment, the colorant layer 18 is formed on an exposed region Re exposed to an outside when the absorbent article 1 is wrapped by the wrapping sheet 10, such that the absorbent article is not easily visible from the outside, thereby a sufficient shielding function is performed. Accordingly, the user can easily carry the absorbent article without wrapping it with a cloth and the user can carry the absorbent article in the similar manner as a person's belongings, for example, pocket tissues or handkerchiefs.

A formation of the colorant layer can be produced, for example, by applying ink or paint to the surface of the wrapping sheet 10. Examples of methods of applying ink include printing, more specifically, gravure printing, flexographic printing, inkjet printing, screen printing, and the like. The colorant layer may have patterns on the wrapping sheet 10, or may have letters or symbols. The color of the colorant (ink or paint) used in forming the colorant layer does not matter. The colorant colors include colors such as red, blue, yellow, black, and the like, as well as white.

The colorant layer formed on the wrapping sheet 10 may be formed on either side of the wrapping sheet 10 as long as the absorbent article is not easily visible from the outside. The colorant layer may also be formed on both sides of the wrapping sheet 10. In the embodiment illustrated in FIGS. 1 to 4, the colorant layer 18 is partially formed on the outer surface side of the wrapping sheet 10, but not on the inner surface side.

Depending on the type and amount of the coloring agent, the coloring agent in the coloring agent layer may easily transfer to the object in contact therewith. However, when the coloring agent layer is not formed on the inner surface side of the wrapping sheet 10 or the amount of the coloring agent layer formed on the inner surface side is reduced as illustrated in the figure, the coloring agent in the coloring agent layer is not easily adhered to the absorbent article 1. In contrast, when the colorant layer is not formed on the outer surface side of the wrapping sheet 10 or the amount of the colorant layer formed on the outer surface side is reduced, it is possible to reduce or prevent the colorant layer from adhering to the object contacting when the wrapped absorbent article is carried.

The colorant layer is formed with an area ratio of greater than 30% and 100% or less in the exposed region Re. In the present specification, the area ratio of the colorant layer (also referred to as the colorant area ratio) refers to the ratio (%) of the area occupied by the applied colorant in a predetermined area. Thus, when the colorant layer is formed in a predetermined area of the wrapping sheet 10 by printing ink, the colorant area ratio is the percentage of the area where printing is applied in that predetermined area. In this case, when the predetermined area is not printed at all, the area ratio is 0%, and when the predetermined area is printed so as to cover the entire area (so-called solid printing), the area ratio is 100%.

When the colorant area ratio in the exposed region Re is 30% or less, the shielding function may be reduced and the contents may be easily visible from the outside. The area ratio of the colorant in the exposed region Re may be preferably 40% or more, more preferably 50% or more, more preferably 65% or more, even more preferably 80% or more, and even more preferably 90% or more.

As described above, the surface on which the colorant layer is formed may be either the outer surface side of the exposed region Re, the inner surface thereof, or both. Therefore, the area ratio of the colorant in the exposed region Re can be a ratio to the area of the entire exposed region Re of the wrapping sheet 10 of the area in plan view of the portion where the colorant layer adhering to the exposed region Re extends, regardless of whether the colorant layer is formed on the outer surface side or the inner surface side of the exposed region Re.

Adjustment of the area ratio of the colorant in the wrapping sheet 10 can be accomplished by adjusting the color density of the colorant throughout a predefined area, or by locally forming and combining a solid printing portion covered 100% by the colorant and a non-colored portion without the colorant. For example, a strip of solid printing portion or non-solid printing portion with a relatively high colorant area portion and a similar strip of non-printed portion may be alternately arranged. Alternatively, small portions (e.g., portions having shapes such as circles, squares, and the like in the plan view) having a solid-printing portion with predetermined area or non-solid printing portion with a relatively high colorant area portion may be locally formed, while other portions may be unprinted.

The wrapping sheet 10 can have the wrapped absorbent article 100 by wrapping the absorbent article 1 such that the first region R1 overlaps the second region R2 (FIGS. 1 to 4). Therefore, when the absorbent article 1 is wrapped with the wrapping sheet 10, the inner surface side of the first region R1 and the outer surface side of the second region R2 at least partially contact with each other. Accordingly, when sealing the edges 13 and 13 of widthwise direction D2 in the wrapping sheet 10, for example, when heat-sealing or ultrasonic sealing is conducted, the inner surface side of the first region R1 and the outer surface side of the second region R2 are usually partially melted and fused together, thereby adhering the inner surface side of the first region R1 and the outer surface side of the second region R2.

However, when the colorant layer is formed between the first region R1 and the second region R2, for example, when the inner surface side of the first region R1 or the outer surface side of the second region R2 or both are printed, in the state where the first region R1 is simply overlapped on the second region R2 (in the state without applying force or heat, and the materials of the first region R1 and the second region R2 are not deformed or denatured), a portion may occur where the material of the inner surface side of the first region R1 cannot directly contact the material of the outer surface side of the second region R2. Therefore, even if the edges 13 and 13 of the widthwise direction D2 are sealed after the wrapping sheet 10 is wrapped in tri-folded to wrap the absorbent article 1, a portion where the sealing property is deteriorated due to the colorant layer may be generated, and the entire seal portions may be deteriorated.

In contrast, in the present embodiment, a portion of the seal portions S and S between the first region R1 and the second region R2 does not have the colorant layer, or has the colorant layer at an area ratio of 30% or less. That is, regardless of whether the colorant is formed on the inner surface side of the first region R1 or the outer surface side of the second region R2, the area ratio in plan view occupied by the colorant present between the first region R1 and the second region R2 is 0 to 30% with respect to the areas of the overlapping seal portions S_{A} and S_{A} (illustrated as meshed area in FIG. 2) in which the first region R1 and the second region R2 directly overlap each other among the seal portions S and S. That is, in the overlapping seal portions S_{A} and S_{A}, there is no colorant layer between the first region R1 and the second region R2, or there is a colorant area ratio of 30% or less.

As described above, in the present embodiment, while the colorant layer 18 is formed in the exposed region Re so that the absorbent article is not easily visible from the outside, the area ratio in the plan view of the colorant layer in the portions where the first region R1 and the second region R2 overlap (overlapping seal portions) S_{A} and S_{A} among the seal portions S and S is 30% or less.
Therefore, the sealing performance of the seal portions S and S can be improved while maintaining the shielding function of the wrapping sheet 10. Further, the design performance is improved, because the colorant layer 18 is disposed in the exposed region Re.

In FIG. 1, the overlapping seal zones S_{A} and S_{A} are indicated as seal zones SZ_{A1} and SZ_{A1} in the first region R1 and as seal zones SZ_{A2} and SZ_{A2} in the second region R2 (both are illustrated in meshed areas). The seal Zone SZ_{A1} is included in the first overlapping region R_{A1}, and the seal zone SZ_{A2} is included in the second overlapping region R_{A2}.

The area ratio of the colorant in the overlapping seal portions S_{A} and S_{A} may be preferably 20% or less, more preferably 15% or less, even more preferably 10% or less, and even more preferably 5% or less. Further, it is further preferable that the colorant layer is not substantially formed in the overlapping seal portions S_{A} and S_{A}. Herein, the colorant layer being not substantially formed refers to the area ratio of the colorant being 1° or less and preferably 0.5% or less.

In addition, in the overlapping seal portions S_{A} and S_{A}, even if the non-solid printing portion or continuous solid printing portion is present on the inner surface side of the first region R1 and the outer surface side of the second region R2, the minimum length of the portion is preferably less than 5 mm and more preferably less than 3 mm.

The area ratio of the colorant may be is partially different in the overlapping seal portions S_{A} and S_{A}. The colorant area ratio of portions S_{E} and S_{E} near the one end 11 thereof in the lengthwise direction D1 of the overlapping seal portions S_{A} and S_{A} (around the tag tape 15) is reduced to be smaller than other portions of the overlapping seal portions S_{A} and S_{A} (see FIG. 2). The portions SE and SE are the portions in which a force is applied when the seal portions S and S are to be opened (unsealed) at first. Therefore, if the sealability of the portions S_{E} and S_{E} are low, the portions SE and SE tend to open. In contrast, the absorbent article 1 is prevented from being unintentionally opened before use, by configuring such that the colorant layer is not present in the portions S_{E} and S_{E}. The above-described portions SE and SE may be portions 5 to 15 mm away from the one end 11 thereof in the lengthwise direction D1.

In the embodiment as illustrated in the figures, the seal portions S and S are formed on the both edges 13 and 13 of the widthwise direction D2 of the wrapping sheet 10, and the overlapping seals SA and SA are also present on the both edges 13 and 13 of the widthwise direction D2 of the wrapping sheet 10, respectively. Each of the overlapping seals S_{A} and S_{A} may have the same or different colorant area ratio. It is preferable that when the both of the colorant area ratio of the overlapping seals S_{A} and S_{A} are the same, the sealability of the seal portions S and S at the both edges 13 and 13 in the widthwise direction D2 become the same, and thus a balance can be achieved. In the present specification, unless otherwise noted, the colorant area ratio in the overlapping seal portions may be the average of the colorant area ratio of the two overlapping seal portions S_{A} and S_{A}.

The colorant area ratio of the overlapping seal portions S_{A} and S_{A} is smaller than that of the exposed region Re. In addition, when the colorant layer is present in the overlapping seal portions S_{A} and S_{A}, the colorant area ratio of the overlapping seal portions S_{A} and S_{A} to the colorant area ratio of the exposed region Re is preferably about 0.001 to 0.8.

In the embodiment illustrated in FIGS. 1 to 4, the colorant layer is not formed on the inner surface side of the wrapping sheet 10. In addition, the colorant layer is not substantially formed on the outer surface side of the overlapping region to which the first region R1 is overlapped in the second region R2, that is, the outer surface side of the second overlapping region RA₂. Therefore, the colorant layer is not substantially formed between the first region R1 and the second region R2 throughout the area where the second region R2 and the first region R1 overlap with the absorbent article 1 being wrapped.

As described above, it is not necessary to divide the area between the seal zone SZ_{A1} and the other region in the first overlapping region R_{A1} or to divide the area between the seal zone SZ_{A2} and the other area in the second overlapping region R_{A2}, when a portion of the seal portions between the first region and the second region does not have the colorant layer, or has the colorant layer at an area ratio of 30% or less, over the entire area where the second region R2 and the first region R1 overlap with the overlapping seal portions SA and SA while the absorbent article 1 is wrapped therein. For example, in the first overlapping region R_{A1}, the forming method or the forming pattern of the colorant layer can be the same in the seal zone SZ_{A1} and the other areas. Also, in the second overlapping region R_{A2}, the forming method or the forming pattern of the colorant layer can be the same in the seal zone SZ_{A2} and the other areas. Accordingly, in the process of manufacturing the wrapping sheet 10, for example, by cutting a rolled sheet after printing on the sheet, when the width of the rolled sheet corresponds to the length of the lengthwise direction D1 of the wrapping sheet 10 and the widthwise direction D2 is set as the conveyance direction when printing, it is easy to maintain the colorant area ratio of the overlapping seal portions S_{A} and S_{A} within the desired area ratio even if a shift occurs in the widthwise direction D2.

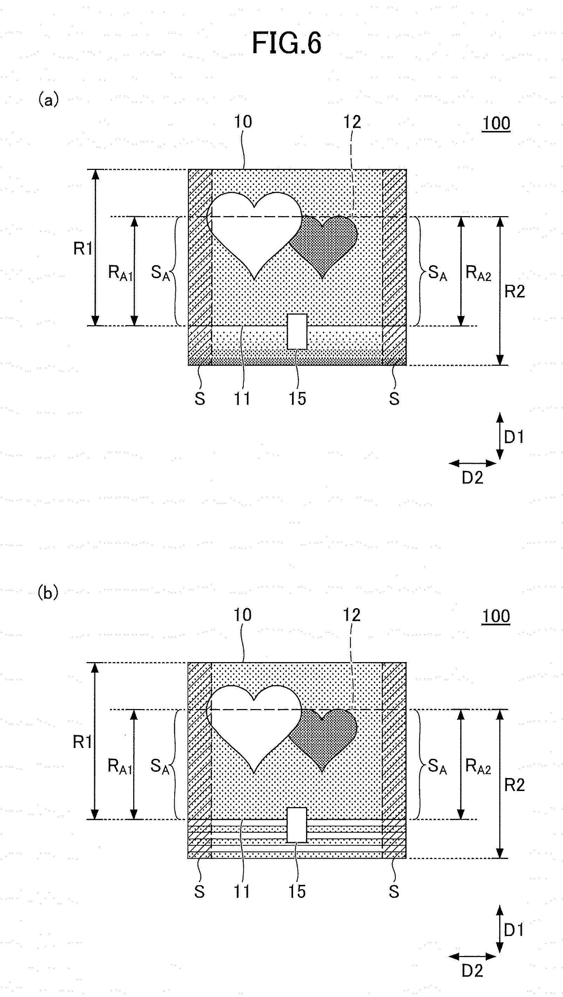

FIGS 6(a) and (b) illustrate modification examples of the wrapped absorbent article 100. The examples illustrated in FIG. 6(a) and (b) are the same as those illustrated in FIG. 2, but the structure of the colorant layer in the region included in the exposed region Re among the second region R2 (the region other than the second overlapping region R_{A2} in the second region R2) is different.

When the colorant area ratio in the second overlapping region R_{A2} is lowered, the sealability at the seal portions is improved. However, the colorant area ratio in the second overlapping region R_{A2} may differ markedly from the colorant area ratio in the first region R1. For example, the colorant layer is not substantially formed in the second overlapping region R_{A2}, while the colorant area ratio in the first region R1 may be, for example, 90%. In such a case, when the overlap position of the first region R1 and the second region R2 is misaligned, the second overlapping region R_{A2} is exposed, and the colorant area ratio of the first region R1 and the colorant area ratio of the second overlapping region R_{A2} appear noticeably unnatural. In particular, when the colorant area ratio near the opposite end 12 in the second overlapping region R_{A2} and the colorant area ratio near the one end 11 in the first region R1 are remarkably different, the difference in the colorant area ratio around the one end 11 in the first region R1 becomes noticeable when the wrapped absorbent article 100 is viewed in a plan view.

In contrast, by configuring the exposed portion of the second region R2 (the region other than the second overlapping region R_{A2} in the second region R2) so that the colorant area ratio includes a portion that gradually or step-wise increases as the colorant area increases in distance becomes further from the opposite end 12 in the lengthwise direction D1, the difference in the colorant area ratio can be made less noticeable. For example, in the example illustrated in FIG. 6(a), the colorant area ratio of the exposed portion of the second region R2 (the region other than the second overlapping region R_{A2} in the second region R2) is gradually increased as the colorant area increases in distance becomes further from the opposite end 12 in the lengthwise direction D1. Thus, in the second region R2, the difference in the colorant area ratio between the second overlapping region R_{A2} and the exposed portion of the second region R2 can be reduced while the colorant area ratio of the exposed region Re can be maintained so that the shielding function is not impaired. Note that the colorant area ratio in FIG. 6(a) is preferably in three steps or more, and more preferably in four steps or more. As the number of steps for increasing the colorant area ratio is increased, the user can visually recognize the colorant area ratio gradually increasing.

In addition, as illustrated in FIG. 6(b), the colorant area ratio of the exposed portion in the second region R2 (the region other than the second overlapping region R_{A2} in the second region R2) can be changed locally. In the present example, the colorant layer in which the colorant layer is not substantially present or absent, or the colorant layer is present 90% or more in the portion, can each be alternately disposed as a strip extending in the widthwise direction D2. The width of the strip portion (the length in the lengthwise direction D1) is preferably in the range of 3 to 7 mm, because the misalignment of the overlap between the first region R1 and the second region R2 is about ±5 mm.

The absorbent article 1 which is wrapped by the wrapping sheet 10 in the present embodiment is sanitary napkins, panty liners, incontinence pads, and the like. A specific configuration may include a liquid-impermeable back sheet, a liquid-permeable top sheet, and a body (the absorbent article body) in which the body is disposed between the two sheets. In the same manner as the wrapping sheet 10, the absorbent article 1 may have an elongated shape in which the first direction (the lengthwise direction of the wrapping sheet 10) D1 and the second direction (the widthwise direction of the wrapping sheet 10) D2 in FIG. 1 are oriented in the lengthwise direction and the widthwise direction, respectively.

As the back sheet of the absorbent article 1, a sheet material having at least a function of liquid non-permeability, such as a sheet of olefin-based resin, for example, polyethylene or polypropylene, may be used. In addition, a laminated non-woven fabric made by laminating a non-woven material with a polyethylene sheet or the like, or a laminated sheet of non-woven fabric that is substantially liquid-impermeable by interposing a waterproof film may be used. In addition, in the viewpoint of preventing an absorbent article from being stuffy, it is further desirable to use a material having moisture permeability.

A top sheet is a liquid-permeable sheet that rapidly permeates body fluids such as menstrual blood, vaginal discharge, urine, and the like. As a top sheet, a porous or non-porous non-woven fabric or porous plastic sheet or the like is preferably used. As the material fibers constituting the non-woven fabric, for example, olefins such as polyethylene, polypropylene, and the like; synthetic fibers such as polyester, polyamide, and the like; regenerated fibers such as rayon, cupra, and the like; blended fibers of these; and natural fibers such as cotton and the like can be used singly or two or more in combination. Alternatively, a composite fiber may be used that combines synthetic fibers with different melting points.

The absorbent material interposed between the back sheet and the top sheet is not particularly limited as long as the absorbent material can absorb and retain bodily fluids. The absorbent material preferably includes cotton pulps and water absorbent polymers. As the water absorbent polymer, superabsorbent polymer (SAP), superabsorbent polymer fiber (SAF), and a combination thereof can be used. Pulps may be a chemical pulp obtained from wood, a cellulosic fiber such as dissolved pulp, or an artificial cellulose fiber such as rayon or acetate. As a raw material for chemical pulp, hardwood, coniferous wood, or the like is used. However, a coniferous material is preferably used, because the fiber length of coniferous material is long. The absorbent article may also be mixed with synthetic fibers.

The thickness of the absorbent article 1 may be in the range of 1 to 20 mm and preferably in the range of 2 to 10 mm. This thickness may not be uniform over the entire surface of the absorbent article 1, and by changing the thickness of the absorbent article, the central portion of the lengthwise direction D1 and the portion corresponding to the grooves in the buttocks may be made of an inflated structure.

As described above, the configuration in which the wrapping sheet is tri-folded in the lengthwise direction (the configuration in which the wrapping sheet is folded at two places) has been mainly described. However, the folding method of the wrapping sheet is not limited to tri-folds. For example, the wrapping sheet may be folded with four or more folds, that is, the wrapping sheet may be folded with three or more places in the lengthwise direction. When an absorbent article is folded with the wrapping sheet, the absorbent article may also be configured to be folded with four folds.

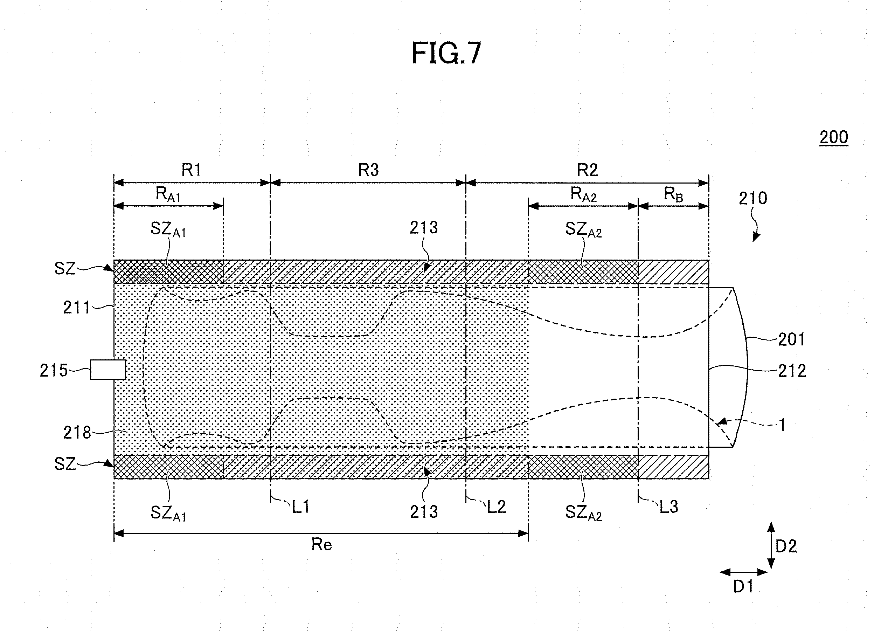

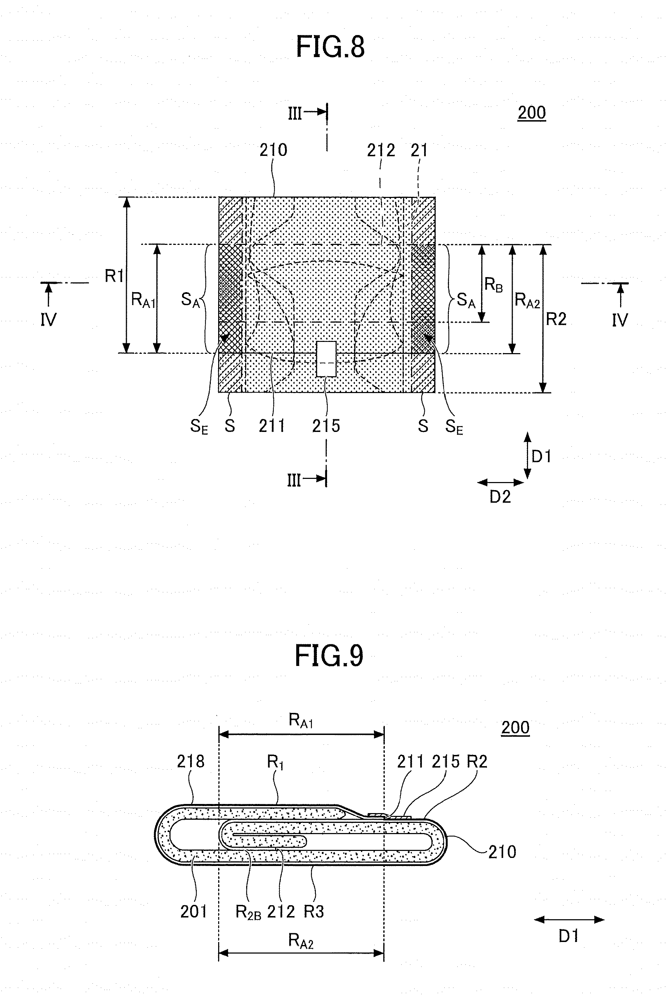

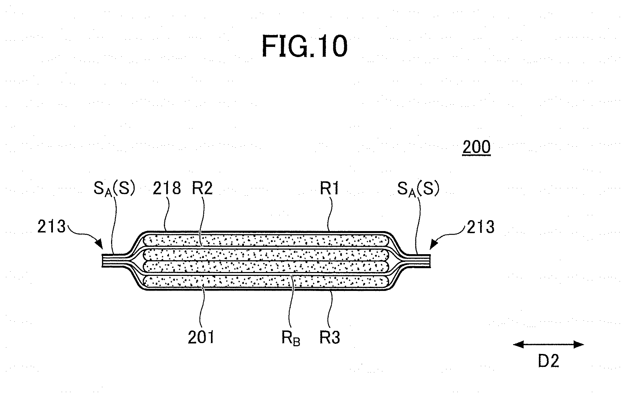

FIG. 7 illustrates a wrapping sheet 210 that is folded in four in the lengthwise direction. In the same manner as FIG. 1, FIG. 7 illustrates a state where an absorbent article 201 is placed on the inner surface side of the wrapping sheet 210, when viewed from outer side of the wrapping sheet. FIG. 7 also illustrates a state in which the wrapped absorbent article 200 is unfolded. Also, FIG. 8 illustrates a state in which the absorbent article 201 is wrapped with the wrapping sheet 210, that is, FIG. 8 illustrates the wrapped absorbent article 200 in a plan view. Further, FIG. 9 illustrates a cross-sectional view of III-III line illustrated in FIG. 8, and FIG. 10 illustrates a cross-sectional view of IV-IV line illustrated in FIG. 8. The cross-sectional view of FIG. 9 corresponds to FIG. 3, and the cross-sectional view of FIG. 10 corresponds to FIG. 4. The configuration illustrated in FIGS. 7 to 10 may be the same configuration as FIGS. 1 to 4, unless otherwise mentioned.

In the same manner as the wrapping sheet 10 (See FIG. 1 etc.), the wrapping sheet 210 can be folded so that the first region R1, which includes the one end 211 in the lengthwise direction D1, can be folded so as to overlap the second region R2, which includes the opposite end 212 in the lengthwise direction D1. That is, the wrapping sheet 210 can be mountain-folded at the second region R2 with the second folding line L2 and then mountain-folded at the first region R1 with the first folding line L1 as illustrated in FIG. 7. However, in the embodiment illustrated in FIG. 7, a portion of the second region R2 is mountain-folded as seen in FIG. 1 in the third folding line L3 before folding in the second folding line L2. More specifically, the folding region R_{B} on the side close to the opposite end 212 in the second region R2 is pre-folded on the side on which the absorbent article 201 is mounted. That is, the wrapping sheet 210 is tri-folded after folding the folding region R_{B}.

The region overlapped with the second region R2 in the first region R1 is designated as the first overlapping region R_{A1}, and the region overlapped with the second region R2 in the second region R2 is designated as the second overlapping region R_{A2}. The first overlapping region R_{A1} is an overlapped region that contacts with the second region R2, and the second overlapping region R_{A2} is an overlapped region that contacts with the first region R1.

After the wrapping sheet 210 and the absorbent article 201 are folded together, the edges 213 and 213 in the widthwise direction D2 that are openably sealed to enable wrapping of the absorbent article, result in forming the seal portions S and S as illustrated in FIGS. 8 and 10 (illustrated by diagonal lines in FIGS. 8 and 10). The seal portions S and S are portions in which a plurality of layers formed by folding the wrapping sheet 210 are bonded to each other. The seal portions S and S in the embodiment illustrated in FIGS. 7 to 10 include an adhesion portion of two layers, an adhesion portion of three layers, and an adhesion portion of four layers.

In the same manner as the wrapped absorbent article 100 (FIGS. 1 to 4), the wrapped absorbent article 200 has a colorant layer 218 formed in the exposed region Re so that the absorbent article is not easily visible from the outside. In contrast, the area ratio in plan view of the colorant area ratio of the colorant layer in the overlapping seal portions S_{A} and S_{A} (FIG. 8 and FIG. 10), which are the overlapping portions of the first region R1 and the second region R2 among the seal portions S and S, is 30% or less. Thus, the sealability of the seal portions S and S can be improved while the shielding function and design of the wrapping sheet 210 are maintained.

In the unfolded wrapping sheet 210, the area in which the seal portions S and S are formed is defined as the seal zone SZ (illustrated by diagonal lines in FIG. 7). The seal zones SZ and SZ extend from the one end 211 to the opposite end 212 in the lengthwise direction D1 at both edges 213 and 213 in the widthwise direction D2 in the wrapping sheet 210, respectively. The overlapping seal zones S_{A} and S_{A} are indicated as zones S2_{A1} and SZ_{A1} in the first region R1 and as zones SZ_{A2} and SZ_{A2} in the second region R2 (both are illustrated as meshed areas).

The color area ratio of the folding region R_{B} in the second region R2 is not particularly limited, but can be the same as that of the colorant area ratio of the second overlapping region R_{A2}. The configuration can be simplified, because the second overlapping region R_{A2} and the folding region R_{B} can be printed at the same colorant area ratio. For example, as illustrated in FIG. 7, the colorant layer may not be substantially formed in the second overlapping region R_{A2} and the folding region R_{B}.

When the sheet is folded with five or more folds, a portion including the one end and/or the opposite end in the lengthwise direction D1 of the wrapping sheet is preferably folded back so that a total of two or more folded lines are formed beforehand, and then tri-folded.

### DESCRIPTION OF THE REFERENCE NUMERALS

1, 201 Absorbent article
10, 210 Wrapping sheet
11, 211 One end of the lengthwise direction
12, 212 Opposite end of the lengthwise direction
15, 215 Tag tape
18, 218 Colorant layer
100, 200 Wrapped absorbent article
D1 Lengthwise direction
D2 Widthwise direction
L1 First folding line
L2 Second folding line
L3 Third folding line
S Seal portion
S_{A} Overlapping seal zone
SD Seal processing portion
SZ Seal zone
SZ_{A1} Seal zone constituting overlapping seal zone S_{A} in the first region R1
SZ_{A2} Seal zone constituting overlapping seal zone S_{A} in the second region R2
R1 First region
R2 Second region
R3 Third region
R_{A1} First overlapping region
R_{A2} Second overlapping region
R_{B} Folding region
Re Exposed region

## Claims

1. A wrapping sheet (10, 210) for an absorbent article (1, 201) which is configured to be wrapped around the absorbent article (1, 201) such that a first region (R1) including one end (11, 211) of the wrapping sheet (10, 210) in a lengthwise direction (D1) overlaps a second region (R2) including an opposite end (12, 212) of the wrapping sheet (10, 210) in the lengthwise direction (D1), and which is provided with seal portions (S) at edges of the wrapping sheet (10, 210) in a widthwise direction (D2) that are openably sealed to enable wrapping of the absorbent article (1, 201),
wherein a colorant layer (18, 218) is formed on an exposed region (Re) exposed to an outside when the absorbent article (1, 201) is wrapped, such that the absorbent article (1, 201) is not easily visible from the outside,
wherein a portion of the seal portions (S) between the first region (R1) and the second region (R2) does not have the colorant layer (18, 218), or has the colorant layer (18, 218) at an area ratio of 30% or less,
wherein the colorant layer (18, 218) is not present in portions (SE and SE) near the one end (11, 211) in the lengthwise direction (D1) of overlapping seal portions (SA and SA), and
wherein the colorant layer (18, 218) is formed with the area ratio of greater than 30% and 100% or less in the exposed region (Re).

2. The wrapping sheet (10, 210) according to claim 1, wherein the seal portion (S) has a width of 5 to 25 mm.

3. The wrapping sheet (10, 210) according to claim 1, wherein the exposed portion of the second region (R2) includes a portion where a colorant layer (18, 218) is formed so that the colorant area ratio gradually or step-wise increases as the colorant area increases in distance becomes further from the opposite end of the wrapping sheet (10, 210) in the lengthwise direction (D1).

4. A wrapped absorbent article (100, 200) comprising a wrapping sheet (10, 210) of claim 1, and an absorbent article (1, 201) wrapped by the wrapping sheet (10, 210).

## Patentansprüche

1. Verpackungsfolie (10, 210) für einen absorbierenden Artikel (1, 201), die dafür ausgelegt ist, so um den absorbierenden Artikel (1, 201) gewickelt zu werden, dass ein erster Bereich (R1), der ein Ende (11, 211) der Verpackungsfolie (10, 210) in einer Längsrichtung (D1) umfasst, sich mit einem zweiten Bereich (R2) überlappt, der ein entgegengesetztes Ende (12, 212) der Verpackungsfolie (10, 210) in der Längsrichtung (D1) umfasst, und der an Rändern der Verpackungsfolie (10, 210) in einer Breitenrichtung (D2) mit Versiegelungsabschnitten (S) versehen ist, die öffnungsfähig versiegelt sind, um ein Umwickeln des absorbierenden Artikels (1, 201) zu ermöglichen,
wobei eine Farbmittelschicht (18, 218) auf einem freiliegenden Bereich (Re) gebildet ist, die nach außen hin freiliegt, wenn der absorbierende Artikel (1, 201) umwickelt ist, so dass der absorbierende Artikel (1, 201) von außen her nicht ohne Weiteres einsehbar ist,
wobei ein Abschnitt der Versiegelungsabschnitte (S) zwischen dem ersten Bereich (R1) und zweiten Bereich (R2) nicht über die Farbmittelschicht (18, 218) verfügt oder die Farbmittelschicht (18, 218) mit einem Flächenverhältnis von 30% oder weniger aufweist,
wobei die Farbmittelschicht (18, 218) in Abschnitten (SE und SE) nahe dem einen Ende (11, 211) in der Längsrichtung (D1) von sich überlappenden Versiegelungsabschnitten (SA und SA) nicht vorhanden ist, und
wobei die Farbmittelschicht (18, 218) mit dem Flächenverhältnis in dem freiliegenden Bereich (Re) von mehr als 30% und 100% oder weniger gebildet ist.

2. Verpackungsfolie (10, 210) nach Anspruch 1,
wobei der Versiegelungsabschnitt (S) eine Breite von 5 bis 25 mm hat.

3. Verpackungsfolie (10, 210) nach Anspruch 1, wobei der freiliegende Abschnitt des zweiten Bereichs (R2) einen Abschnitt umfasst, in dem eine Farbmittelschicht (18, 218) so gebildet ist, dass das Farbmittelflächenverhältnis graduell oder stufenweise zunimmt, wenn die Farbmittelfläche im Abstand zunimmt sich in der Längsrichtung (D1) vom entgegengesetzten Ende der Verpackungsfolie (10, 210) weiter entfernt.

4. Umwickelter absorbierender Artikel (100, 200) mit einer Verpackungsfolie (10, 210) nach Anspruch 1 und einem absorbierenden Artikel (1, 201), der mit der Verpackungsfolie (10, 210) umwickelt ist.

## Revendications

1. Feuille d'emballage (10, 210) pour un article absorbant (1, 201), adaptée pour être enroulée autour de l'article absorbant (1, 201) de telle sorte qu'une première zone (R1) comprenant une extrémité (11, 211) de la feuille d'emballage (10, 210) dans une direction longitudinale (D1) chevauche une deuxième zone (R2) comprenant une extrémité opposée (12, 212) de la feuille d'emballage (10, 210) dans la direction longitudinale (D1), et qui est pourvue, sur des bords de la feuille d'emballage (10, 210) dans une direction de la largeur (D2), de sections de scellement (S) qui sont scellées de manière à pouvoir être ouvertes pour permettre l'enveloppement de l'article absorbant (1, 201),
sachant qu'une couche de colorant (18, 218) est formée sur une zone exposée (Re) qui est exposée à l'extérieur lorsque l'article absorbant (1, 201) est enveloppé, de sorte que l'article absorbant (1, 201) n'est pas facilement visible de l'extérieur,
sachant qu'une section des sections de scellement (S) située entre la première zone (R1) et la deuxième zone (R2) ne possède pas la couche de colorant (18, 218) ou possède la couche de colorant (18, 218) avec un rapport de surface de 30% ou moins,
sachant que la couche de colorant (18, 218) n'est pas présente dans des sections (SE et SE) proches d'une extrémité (11, 211) dans la direction longitudinale (D1) de sections de scellement (SA et SA) qui se chevauchent, et
sachant que la couche de colorant (18, 218) est formée de telle sorte que le rapport de surface est supérieur à 30% et égal ou inférieur à 100% dans la zone exposée (Re).

2. La feuille d'emballage (10, 210) selon la revendication 1,
sachant que la section de scellement (S) a une largeur de 5 à 25 mm.

3. La feuille d'emballage (10, 210) selon la revendication 1, sachant que la section exposée de la deuxième zone (R2) comprend une section dans laquelle une couche de colorant (18, 218) est formée de telle sorte que le rapport de surface de colorant augmente progressivement ou par étapes à mesure que la surface de colorant augmente en distance s'éloigne davantage dans la direction longitudinale (D1) de l'extrémité opposée de la feuille d'emballage (10, 210) .

4. Article absorbant enveloppé (100, 200) comprenant une feuille d'emballage (10, 210) selon la revendication 1 et un article absorbant (1, 201) enveloppé par la feuille d'emballage (10, 210).
